(19) Europäisches Patentamt
European Patent Office
Office européen des brevets

(11) Veröffentlichungsnummer: **0 123 700 A1**

(12) # EUROPÄISCHE PATENTANMELDUNG

(21) Anmeldenummer: **83104094.4**

(22) Anmeldetag: **27.04.83**

(51) Int. Cl.³: **C 07 D 213/79**, C 07 D 213/81, A 61 K 31/44

(43) Veröffentlichungstag der Anmeldung: **07.11.84 Patentblatt 84/45**

(84) Benannte Vertragsstaaten: **AT BE FR GB IT LU NL SE**

(71) Anmelder: **Byk Gulden Lomberg Chemische Fabrik GmbH, Byk-Gulden-Strasse 2, D-7750 Konstanz (DE)**

(72) Erfinder: **Amschler, Hermann, Dr., Hohenhewenstrasse 19, D-7760 Radolfzell (DE)**
Erfinder: **Sanders, Karl, Dr., Hinterhauser Strasse 2, D-7750 Konstanz (DE)**
Erfinder: **Ulrich, Wolf-Rüdiger, Dr., Brandesstrasse 14, D-7750 Konstanz (DE)**

(54) **Substituierte Picolinsäuren, Verfahren zu ihrer Herstellung, ihre Verwendung und sie enthaltende Arzneimittel.**

(57) Substituierte Picolinsäuren der allgemeinen Formel I

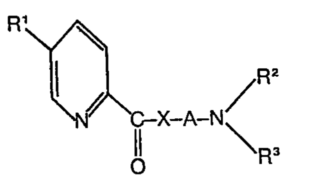

(I),

worin $R^1$ Alkyl mit 1 bis 7 Kohlenstoffatomen – jedoch nicht Butyl, wenn $R^2$ und $R^3$ die Bedeutung Alkyl haben und X NH bedeutet –, Cycloalkylalkyl mit 3 bis 7 Kohlenstoffatomen im Cycloalkyl- und 1 bis 4 Kohlenstoffatomen im Alkylenteil, Phenyl oder substituiertes Phenyl mit bis zu drei Substituenten aus der Gruppe Halogen, Hydroxy, Alkyl mit 1 bis 4 Kohlenstoffatomen und Alkoxy mit 1 bis 4 Kohlenstoffatomen bedeutet, $R^2$ Alkyl mit 1 bis 4 Kohlenstoffatomen oder Alkenyl mit 3 bis 5 Kohlenstoffatomen bedeutet, $R^3$ Alkyl mit 1 bis 4 Kohlenstoffatomen oder Alkenyl mit 3 bis 5 Kohlenstoffatomen bedeutet, oder $R^2$ und $R^3$ gemeinsam und unter Einschluß des Stickstoffatoms, an das sie gebunden sind, Pyrrolidino, Morpholino, Piperidino, Hexahydroazepino oder in 4-Position durch $R^4$ substituiertes 1-Piperazinyl bedeuten, $R^4$ Wasserstoff, Alkyl mit 1 bis 4 Kohlenstoffatomen, Phenyl, Benzyl oder substituiertes Phenyl mit bis zu drei Substituenten aus der Gruppe Halogen, Hydroxy, Alkyl mit 1 bis 4 Kohlenstoffatomen und Alkoxy mit 1 bis 4 Kohlenstoffatomen bedeutet, A geradkettiges oder verzweigtes Alkylen mit 1 bis 5 Kohlenstoffatomen und X O (Sauerstoff) oder NH bedeutet, sowie die Säureadditionssalze dieser Verbindungen, wobei $R^1$, $R^2$ und $R^3$ nicht gleichzeitig Alkyl bedeuten, wenn X O (Sauerstoff) und A Ethylen darstellt, sind neue Verbindungen mit blutdrucksenkender Wirksamkeit. Verfahren zur Herstellung dieser Verbindungen werden angegeben.

EP 0 123 700 A1

# Substituierte Picolinsäuren, Verfahren zu ihrer Herstellung, ihre Verwendung und sie enthaltende Arzneimittel

## Anwendungsgebiet der Erfindung

Die Erfindung betrifft substituierte Picolinsäuren, Verfahren zu ihrer Herstellung, ihre Verwendung und sie enthaltende Arzneimittel.

Die erfindungsgemäßen Verbindungen werden in der pharmazeutischen Industrie als Zwischenprodukte und zur Herstellung von Medikamenten verwendet.

## Bekannter technischer Hintergrund

In der Deutschen Offenlegungsschrift DE-OS 23 06 671 werden di- und trisubstituierte Picolinsäure- und Thio-picolinsäurederivate als Dopamin-β-Hydroxylasehemmer mit antihypertensiver Wirkung beschrieben.- In den US-Patenten USP 4,044,140 und USP 4,127,662 werden Triphenylmethyl-substituierte Picolinsäurederivate beschrieben, die als Anti-Aknemittel Verwendung finden sollen. - Die Japanische Offenlegungsschrift 50 - 130 770 offenbart (Halogen)butyl-substituierte Picolinsäureamide, die eine hypotensive Wirkung besitzen sollen. - In der sowjetischen Patentschrift SU 598 892 wird ein n-Butyl-picolinsäureamid beschrieben, das als Antiarrhythmicum eingesetzt werden soll. - Im britischen Patent GB 1,037,380 werden Pyridincarbonsäurederivate beansprucht, die in pharmazeutischen Produkten Verwendung finden sollen.

Es wurde nun eine Klasse neuer substituierter Picolinsäuren synthetisiert, die weder in den erwähnten Publikationen genannt noch durch sie nahegelegt werden. Ferner wurde gefunden, daß diese substituierten Picolinsäuren interessante, besonders vorteilhafte pharmakologische Eigenschaften aufweisen.

## Beschreibung der Erfindung

Gegenstand der Erfindung sind substituierte Picolinsäuren der allgemeinen Formel I,

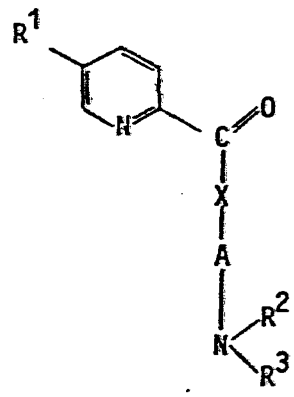

(I),

worin

$R^1$ Alkyl mit 1 bis 7 Kohlenstoffatomen - jedoch nicht Butyl, wenn $R^2$ und $R^3$ die Bedeutung Alkyl haben und X NH bedeutet -, Cycloalkylalkyl mit 3 bis 7 Kohlenstoffatomen im Cycloalkyl- und 1 bis 4 Kohlenstoffatomen im Alkylenteil, Phenyl oder substituiertes Phenyl mit bis zu drei Substituenten aus der Gruppe Halogen, Hydroxy, Alkyl mit 1 bis 4 Kohlenstoffatomen und Alkoxy mit 1 bis 4 Kohlenstoffatomen bedeutet,

$R^2$ Alkyl mit 1 bis 4 Kohlenstoffatomen oder Alkenyl mit 3 bis 5 Kohlenstoffatomen bedeutet,

$R^3$ Alkyl mit 1 bis 4 Kohlenstoffatomen oder Alkenyl mit 3 bis 5 Kohlenstoffatomen bedeutet, oder

$R^2$ und $R^3$ gemeinsam und unter Einschluß des Stickstoffatoms, an das sie gebunden sind, Pyrrolidino, Morpholino, Piperidino, Hexahydroazepino oder in 4-Position durch $R^4$ substituiertes 1-Piperazinyl bedeuten,

$R^4$ Wasserstoff, Alkyl mit 1 bis 4 Kohlenstoffatomen, Phenyl, Benzyl oder substituiertes Phenyl mit bis zu drei Substituenten aus der Gruppe Halogen, Hydroxy, Alkyl mit 1 bis 4 Kohlenstoffatomen und Alkoxy mit 1 bis 4 Kohlenstoffatomen bedeutet,

A geradkettiges oder verzweigtes Alkylen mit 1 bis 5 Kohlenstoffatomen und

X O (Sauerstoff) oder NH bedeutet,

sowie die Säureadditionssalze dieser Verbindungen, wobei $R^1$, $R^2$ und $R^3$ nicht gleichzeitig Alkyl bedeuten, wenn X O(Sauerstoff) und A Ethylen darstellt.

Alkylreste können geradkettig (z.B. Methyl, Ethyl, Propyl, Pentyl, Hexyl und Heptyl) oder verzweigt (z.B. Isopropyl, Isobutyl, sec.-Butyl, tert.-Butyl, Neopentyl, 3-Methylbutyl oder 3,3-Dimethylbutyl) sein.

Cycloalkylreste der Cycloalkylalkylgruppen sind der Cyclopropyl-, Cyclobutyl-, Cycloheptyl- und insbesondere der Cyclopentyl- und Cyclohexylrest. Alkylenreste der Cycloalkylalkylgruppen sind der Tetramethylen-, Trimethylen-, Ethylen- und insbesondere der Methylenrest. Ein bevorzugter Cycloalkylalkylrest ist der Cyclohexylmethylrest.

Halogenatome im Sinne der Erfindung sind das Fluor-, Chlor- und Bromatom.

Alkoxygruppen sind beispielsweise die Butoxy-, Propoxy- und insbesondere die Ethoxy- und die Methoxygruppe.

Als Alkenylreste seien beispielsweise der Allyl-, der 2-Methylallyl- und der 1-Butenylrest genannt.

Als Alkylengruppen seien beispielsweise die Methylen-, Ethylmethylen-, Propylen-, Tetramethylen- und Pentamethylen-, insbesondere die Trimethylen- und Ethylengruppe genannt.

Als Salze kommen alle Säureadditionssalze in Betracht. Besonders erwähnt seien die pharmakologisch verträglichen Salze der in der Galenik üblicherweise verwendeten anorganischen und organischen Säuren. Pharmakologisch unverträgliche Salze, die beispielsweise bei der Herstellung der erfindungsgemäßen Verbindungen im industriellen Maßstab als Verfahrensprodukte zunächst anfallen können, werden durch dem Fachmann bekannte Verfahren in pharmakologisch verträgliche Salze übergeführt. Als solche eignen sich beispielsweise in Wasser leicht oder auch schwer lösliche Säureadditionssalze, wie das Hydrochlorid, Hydrobromid, Hydroiodid, Phosphat, Nitrat, Sulfat, Acetat, Citrat, Gluconat, Benzoat, Hibenzat (2-(4-Hydroxybenzoyl)-benzoat), Propionat, Butyrat, Sulfosalicylat, Maleat, Laurat, Malat, Fumarat, Succinat, Oxalat, Tartrat, Amsonat (4,4'-Diamino-stilben-2,2'-disulfonat), Embonat (1,1'-Methylen-bis-2-hydroxy-3-naphthoat), Metembonat, Stearat, Tosilat (p-Toluolsulfonat), 2-Hydroxy-3-naphthoat, 3-Hydroxy-2-naphthoat, Mesilat (Methansulfonat), ferner Salze mit Bumetanid (3-(Butylamino)-4-phenoxy-5-sulfamoyl-benzoesäure), Furosemid (4-Chlor-N-furfuryl-5-sulfamoylanthranilsäure), Azosemid [2-Chlor-5-(1H-tetrazol-5-yl)-N$^{1}$-2-thenylsulfanil-amid], Galosemid [N-[4-(3-Trifluormethyl-

anilino)-3-pyridylsulfonyl]-propionamid], Besunid (4-Benzyl-3-(butylamino)-5-sulfamoyl-benzoesäure), Piretanid (4-Phenoxy-3-(1-pyrrolidinyl)-5-sulfamoyl-benzoesäure), Etacrynsäure ([2,3-Dichlor-4-(2-methylenbutyryl)-phenoxy]-essigsäure), Tienilinsäure ([2,3-Dichlor-4-(2-thenoyl)-phenoxy]-essigsäure), 4-Chlor-3-sulfamoyl-benzoesäure.

Eine Ausgestaltung (Ausgestaltung a) der Erfindung sind Verbindungen der allgemeinen Formel I, worin $R^1$ Alkyl mit 1 bis 7 Kohlenstoffatomen -jedoch nicht Butyl, wenn $R^2$ und $R^3$ die Bedeutung Alkyl haben und X NH darstellt- bedeutet, $R^2$ Alkyl mit 1 bis 4 Kohlenstoffatomen oder Alkenyl mit 3 bis 5 Kohlenstoffatomen bedeutet, $R^3$ eine der Bedeutungen von $R^2$ hat oder $R^2$ und $R^3$ gemeinsam und unter Einschluß des Stickstoffatoms, an das sie gebunden sind, Pyrrolidino, Morpholino, Piperidino, Hexahydroazepino oder in 4-Position durch $R^4$ substituiertes 1-Piperazinyl bedeuten, $R^4$ Wasserstoff, Alkyl mit 1 bis 4 Kohlenstoffatomen, Phenyl, Benzyl oder substituiertes Phenyl mit bis zu drei Substituenten aus der Gruppe Halogen, Hydroxy, Alkyl mit 1 bis 4 Kohlenstoffatomen und Alkoxy mit 1 bis 4 Kohlenstoffatomen bedeutet, A geradkettiges oder verzweigtes Alkylen mit 1 bis 5 Kohlenstoffatomen und X O(Sauerstoff) oder NH bedeutet, sowie die Säureadditionssalze dieser Verbindungen, wobei $R^2$ und $R^3$ nicht gleichzeitig Alkyl bedeuten, wenn X O(Sauerstoff) und A Ethylen darstellt.

Eine weitere Ausgestaltung (Ausgestaltung b) der Erfindung sind Verbindungen der allgemeinen Formel I, worin $R^1$ Cycloalkylalkyl mit 3 bis 7 Kohlenstoffatomen im Cycloalkyl- und 1 bis 4 Kohlenstoffatomen im Alkylenteil, Phenyl oder substituiertes Phenyl mit bis zu drei Substituenten aus der Gruppe Halogen, Hydroxy, Alkyl mit 1 bis 4 Kohlenstoffatomen und Alkoxy mit 1 bis 4 Kohlenstoffatomen bedeutet, $R^2$ Alkyl mit 1 bis 4 Kohlenstoffatomen oder Alkenyl mit 3 bis 5 Kohlenstoffatomen bedeutet, $R^3$ eine der Bedeutungen von $R^2$ hat, A geradkettiges oder verzweigtes Alkylen mit 1 bis 5 Kohlenstoffatomen und X O(Sauerstoff) oder NH bedeutet, sowie die Säureadditionssalze dieser Verbindungen.

Eine weitere Ausgestaltung (Ausgestaltung c) der Erfindung sind Verbindungen der allgemeinen Formel I, worin $R^1$ Cycloalkylalkyl mit 3 bis 7 Kohlenstoffatomen im Cycloalkyl- und 1 bis 4 Kohlenstoffatomen im Alkylenteil, Phenyl oder substituiertes Phenyl mit bis zu drei Substituenten aus der Gruppe Halogen, Hydroxy, Alkyl mit 1 bis 4 Kohlenstoffatomen und Alkoxy mit 1 bis 4 Kohlenstoffatomen bedeutet, $R^2$ und $R^3$ gemeinsam und unter Einschluß des Stickstoffatoms, an das sie gebunden sind, Pyrrolidino, Morpholino, Piperidino, Hexahydroazepino oder in 4-Position durch $R^4$ substituiertes 1-Piperazinyl bedeuten, $R^4$ Wasserstoff, Alkyl mit 1 bis 4 Kohlenstoffatomen, Phenyl, Benzyl oder substituiertes Phenyl mit bis zu drei Substituenten aus der Gruppe Halogen, Hydroxy, Alkyl mit 1 bis 4 Kohlenstoffatomen und Alkoxy mit 1 bis 4 Kohlenstoffatomen bedeutet, A geradkettiges oder verzweigtes Alkylen mit 1 bis 5 Kohlenstoffatomen und X O(Sauerstoff) oder NH bedeutet, sowie die Säureadditionssalze dieser Verbindungen.

Bevorzugte Verbindungen der Ausgestaltung a sind solche der allgemeinen Formel I*

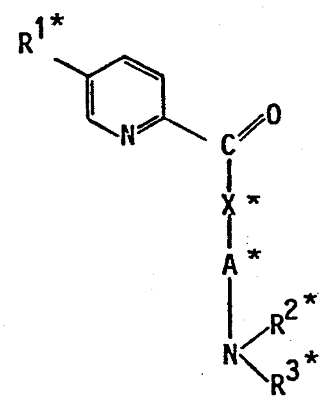

(I*),

worin

$R^{1*}$ Alkyl mit 1 bis 5 Kohlenstoffatomen (jedoch nicht Butyl, wenn $R^{2*}$ und $R^{3*}$ die Bedeutung Methyl oder Ethyl haben und X NH darstellt) bedeutet,

$R^{2*}$ Methyl oder Ethyl bedeutet,

$R^{3*}$ Methyl oder Ethyl bedeutet oder

$R^{2*}$ und $R^{3*}$ gemeinsam und unter Einschluß des Stickstoffatoms, an das sie gebunden sind, Pyrrolidino, Piperidino oder in 4-Position durch $R^{4*}$ substituiertes 1-Piperazinyl bedeuten,

$R^{4*}$ Phenyl oder durch Methoxy oder Ethoxy substituiertes Phenyl bedeutet,

A* Ethylen oder Trimethylen und

X* O(Sauerstoff) oder NH bedeutet,

sowie die Säureadditionssalze dieser Verbindungen, wobei $R^{2*}$ und $R^{3*}$ nicht Methyl oder Ethyl bedeuten, wenn X* O(Sauerstoff) und A* Ethylen darstellt.

Bevorzugte Verbindungen der Ausgestaltung b sind solche der allgemeinen Formel I**

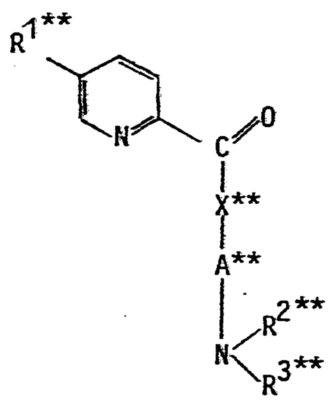

(I**),

worin

$R^{1**}$ Cycloalkylalkyl mit 5 oder 6 Kohlenstoffatomen im Cycloalkyl- und 1 oder 2 Kohlenstoffatomen im Alkylenteil, Phenyl oder substituiertes Phenyl mit bis zu zwei Substituenten aus der Gruppe Chlor, Methoxy oder Ethoxy bedeutet,

$R^{2**}$ Methyl oder Ethyl bedeutet,

$R^{3**}$ Methyl oder Ethyl bedeutet,

A** Ethylen oder Trimethylen und

X** O(Sauerstoff) oder NH bedeutet,

sowie die Säureadditionssalze dieser Verbindungen.

Bevorzugte Verbindungen I** sind solche, in denen A** Trimethylen und X** NH bedeutet und $R^{1**}$, $R^{2**}$ und $R^{3**}$ die oben angegebenen Bedeutungen haben, sowie die pharmakologisch verträglichen Säureadditionssalze dieser Verbindungen.

Besonders bevorzugte Verbindungen I** sind solche, in denen $R^{1**}$ Cyclohexylmethyl, Phenyl oder 4-Chlorphenyl bedeutet, $R^{2**}$ und $R^{3**}$ Methyl oder Ethyl bedeuten, A** Trimethylen und X** NH darstellt, sowie die pharmakologisch verträglichen Säureadditionssalze dieser Verbindungen.

Bevorzugte Verbindungen der Ausgestaltung c sind solche der allgemeinen Formel I***

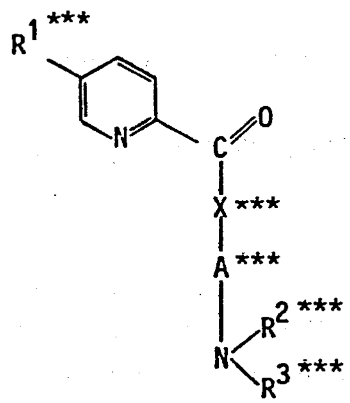

(I***),

worin

$R^{1***}$ Cycloalkylalkyl mit 5 oder 6 Kohlenstoffatomen im Cycloalkyl- und 1 oder 2 Kohlenstoffatomen im Alkylenteil, Phenyl oder substituiertes Phenyl mit bis zu zwei Substituenten aus der Gruppe Chlor, Methoxy oder Ethoxy bedeutet,

$R^{2***}$ und $R^{3***}$ gemeinsam und unter Einschluß des Stickstoffatoms, an das sie gebunden sind, Pyrrolidino, Piperidino oder in 4-Position durch $R^{4***}$ substituiertes 1-Piperazinyl bedeuten,

$R^{4***}$ Wasserstoff, Methyl, Phenyl oder durch Methoxy oder Ethoxy substituiertes Phenyl bedeutet,

A*** Ethylen oder Trimethylen und

X*** O(Sauerstoff) oder NH bedeutet,

sowie die Säureadditionssalze dieser Verbindungen.

Bevorzugte Verbindungen I*** sind solche, in denen A*** Trimethylen und X*** NH bedeutet und $R^{1***}$, $R^{2***}$, $R^{3***}$ und $R^{4***}$ die oben angegebenen Bedeutungen haben, sowie die pharmakologisch verträglichen Säureadditionssalze dieser Verbindungen.

Besonders bevorzugte Verbindungen I*** sind solche, in denen $R^{1***}$ Cyclohexylmethyl, Phenyl oder 4-Chlorphenyl bedeutet, $R^{2***}$ und $R^{3***}$ gemeinsam und unter Einschluß des Stickstoffatoms, an das sie gebunden sind, Piperidino oder in 4-Position durch $R^{4***}$ substituiertes 1-Piperazinyl bedeuten, $R^{4***}$ 2-Methoxy- oder 2-Ethoxy-substituiertes Phenyl bedeutet, A*** Trimethylen und X*** NH darstellt, sowie die pharmakologisch verträglichen Säureadditionssalze dieser Verbindungen.

Die Ausgestaltungen b und c sind gegenüber der Ausgestaltung a bevorzugt.

Als Vertreter der erfindungsgemäßen Verbindungen seien beispielsweise genannt:

5-n-Propylpyridin-2-carbonsäure-(3-diethylaminopropyl)ester
5-Ethylpyridin-2-carbonsäure-(5-dimethylaminopentyl)amid
5-n-Hexylpyridin-2-carbonsäure-(2-diethylaminoethyl)amid
5-Isopropylpyridin-2-carbonsäure-(3-dimethylaminobutyl)amid
5-n-Butylpyridin-2-carbonsäure-(3-piperidinopropyl)amid
5-n-Hexylpyridin-2-carbonsäure-(2-pyrrolidinoethyl)amid
5-Ethylpyridin-2-carbonsäure-(5-morpholinopentyl)ester
5-Methylpyridin-2-carbonsäure-(2-piperidinobutyl)amid
5-Cyclopentylmethylpyridin-2-carbonsäure-(3-diethylaminopropyl)ester
5-(2-Cyclohexylethyl)-pyridin-2-carbonsäure-(4-dimethylaminobutyl)amid
5-Cycloheptylmethylpyridin-2-carbonsäure-(3-diethylaminopropyl)amid
5-(3-Cyclobutylpropyl)-pyridin-2-carbonsäure-(2-diisopropylaminoethyl)ester
5-Cyclopropylmethylpyridin-2-carbonsäure-(3-pyrrolidinopropyl)amid
5-Cyclohexylmethylpyridin-2-carbonsäure-(3-[4-(2-methylphenyl)piperazinyl-1-]-propyl)amid
5-(2-Cyclopentylethyl)-pyridin-2-carbonsäure-[3-(4-phenylpiperazinyl-1)-propyl]amid
5-Cyclohexylmethylpyridin-2-carbonsäure-(4-[4-(2-methoxyphenyl)piperazinyl-1]-butyl)amid

5-(3-Cyclobutylpropyl)-pyridin-2-carbonsäure-(3-[4-(2-methoxyphenyl)-piperazinyl-1]-propyl)amid
5-(4-Chlorphenyl)-pyridin-2-carbonsäure-(4-diethylaminobutyl)amid
5-(2,4-Dichlorphenyl)-pyridin-2-carbonsäure- (3-dimethylaminopropyl)amid
5-(4-Hydroxyphenyl)-pyridin-2-carbonsäure-(4-diisopropylaminobutyl)ester
5-(4-Ethoxyphenyl)-pyridin-2-carbonsäure-(2-diethylaminopropyl)amid
5-(2,4-Dichlorphenyl)-pyridin-2-carbonsäure-(3-[4-(2-methoxyphenyl)-piperazinyl-1]-propyl)amid
5-(4-Chlorphenyl)-pyridin-2-carbonsäure-[3-(4-methylpiperazinyl-1)-propyl]-amid
5-Phenylpyridin-2-carbonsäure-(4-[4-(2-methoxyphenyl)piperazinyl-1]-butyl)-amid
5-(4-Hydroxyphenyl)-pyridin-2-carbonsäure-(3-[4-(2-methylphenyl)piperazinyl-1]-propyl)amid
5-(2,4,6-Trichlorphenyl)-pyridin-2-carbonsäure-(3-[4-(2-methoxyphenyl)-piperazinyl-1]-propyl)amid,
insbesondere
5-Cyclohexylmethylpyridin-2-carbonsäure-(3-[4-(2-methoxyphenyl)-piperazinyl-1]-propyl)-amid
5-(4-Chlorphenyl)-pyridin-2-carbonsäure-(3-diethylaminopropyl)-amid
5-(4-Chlorphenyl)-pyridin-2-carbonsäure-(3-[4-(2-methoxyphenyl)-piperazinyl-1]-propyl)-amid
5-Phenylpyridin-2-carbonsäure-(3-[4-(2-methoxyphenyl)-piperazinyl-1]-propyl)-amid
sowie die Säureadditionssalze dieser Verbindungen.

Wenn in der nachfolgenden Beschreibung von den Verbindungen "der (allgemeinen) Formel I" gesprochen wird, so umfaßt dieser Ausdruck (bzw. er steht stellvertretend für) die Verbindungen der Ausgestaltungen a, b und c, die Verbindungen I*, I** und I***, ihre bevorzugten und besonders bevorzugten Vertreter sowie die expressis verbis genannten Verbindungen; darüberhinaus umfaßt dieser Ausdruck die Säureadditionssalze dieser Verbindungen.

Ein weiterer Gegenstand der Erfindung ist ein Verfahren zur Herstellung von substituierten Picolinsäuren der allgemeinen Formel I, das dadurch gekennzeichnet ist, daß man Picolinsäurederivate der allgemeinen Formel II

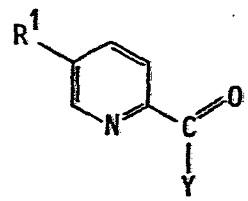

(II),

mit Aminen der allgemeinen Formel III

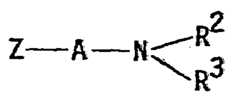

(III),

umsetzt und gewünschtenfalls anschließend in die Säureadditionssalze überführt, wobei die Reste Y und Z so zu wählen sind, daß das gewünschte Glied X gebildet wird, und wobei $R^1$, $R^2$, $R^3$, A und X die oben angegebenen Bedeutungen haben.

Welche Reste Y und Z gewählt werden müssen, ist dem Fachmann aufgrund seines Fachwissens geläufig. Die folgende Aufstellung gibt einen Überblick über geeignete Reste Y und Z bei gewünschtem X:

X = O (Sauerstoff)

a) Y = OH, Z = OH (Veresterung)

b) Y = Halogen, Z = OH

c) Y = OR' (R' ist z.B. Methyl, Ethyl), Z = OH (Umesterung)

d) Y = $O^{\ominus}$ (Salz eines Picolinsäurederivates), Z = Abgangsgruppe (z.B. Halogen, wie Brom oder Chlor) (Alkylierung)

X = NH

e) Y = OH, Z = $NH_2$

f) Y = Halogen, Z = $NH_2$

g) Y = OR' (R' ist z.B. Methyl, Ethyl), Z = $NH_2$

h) Y = -O-CO-OR' (R' ist z.B. Methyl, Ethyl), Z = $NH_2$

Die Umsetzung nach a) wird analog zu üblichen Veresterungsreaktionen durchgeführt, vorzugsweise in einem wasserfreien Lösungsmittel (z.B. Dichlorethan, Toluol, Benzol, Dioxan), unter saurer Katalyse (Mineralsäure, saurer Ionenaustauscher), bei Temperaturen zwischen 50 und 150°C, vorteilhafterweise bei der Siedetemperatur des verwendeten Lösungsmittels, und unter Verwendung eines Wasserabscheiders oder eines wasserbindenden Molekularsiebes.

Die Umsetzung nach b) wird zweckmäßigerweise in einem inerten, wasserfreien Lösungsmittel durchgeführt (z.B. Acetonitril, Dichlorethan, Tetrahydrofuran, Benzol), wobei als Säurehalogenid bevorzugt ein Säurebromid oder - chlorid verwendet wird. Je nach Reaktivität des eingesetzten Alkohols erfolgt die Umsetzung unter gleichzeitiger Kühlung oder unter leichtem Erwärmen, bevorzugt jedoch bei Raumtemperatur. Die Reaktion wird unter Zusatz eines säurebindenden Mittels (z.B. eines Amins, wie Triethylamin) oder mit einem Überschuß an Verbindung III durchgeführt.

Die Umesterungsreaktion nach c) erfolgt vorzugsweise unter Basenkatalyse, z.B. durch Zusatz katalytischer Mengen an Natrium, in inerten, hochsiedenden Lösungsmitteln (z.B. Xylol) oder auch ohne Lösungsmittel, bei Temperaturen zwischen 50 und 180°C.

Die Alkylierungsreaktion nach d) wird entweder als Einphasenreaktion (direkte Umsetzung des Picolinsäuresalzes mit dem Alkylierungsmittel) oder als Zweiphasenreaktion unter Zusatz eines Phasentransferkatalysators (Einsatz der freien Säure und einer geeigneten Base) vorgenommen.

Die Amidbildungsreaktion nach e) erfolgt durch Erhitzen (vorzugsweise auf 50 bis 100°C) der entsprechenden Säure und des Amins in inerten, wasserfreien Lösungsmitteln (wie z.B. Tetrahydrofuran, Dioxan, Toluol), vorteilhafterweise in Gegenwart eines wasserbindenden Mittels (z.B. eines Carbodiimids).

Die Umsetzung nach f) kann analog der unter b) beschriebenen Reaktion durchgeführt werden. Die Umsetzung nach g) erfolgt vorteilhafterweise wie bei c) beschrieben ohne Zusatz eines Lösungsmittels bei erhöhter Temperatur, gegebenenfalls unter Basenkatalyse.

Die Umsetzung mit dem gemischten Anhydrid nach h) erfolgt vorzugsweise in wasserfreien Lösungsmitteln (z.B. in Dichlormethan, Chloroform, Dioxan) bei Temperaturen zwischen $0^o$ und $50^oC$, bevorzugt bei Raumtemperatur. Statt eines gemischten Anhydrides kann natürlich auch das reine Anhydrid der entsprechenden Picolinsäure verwendet werden.

Säureadditionssalze erhält man durch Auflösen der freien Base in einem geeigneten Lösungsmittel, z.B. in einem chlorierten Kohlenwasserstoff, wie Methylenchlorid oder Chloroform, oder einem niedermolekularen aliphatischen Alkohol (Ethanol, Isopropanol), das die gewünschte Säure enthält, oder dem die gewünschte Säure anschließend zugegeben wird.

Die Salze werden durch Filtrieren, Umfällen, Ausfällen mit einem Nichtlösungsmittel für das Anlagerungssalz oder durch Verdampfen des Lösungsmittels gewonnen.

Erhaltene Salze können durch Alkalisierung, z.B. mit wäßrigem Natriumhydrogencarbonat, in die freien Basen umgewandelt werden, welche wiederum in Säureadditionssalze übergeführt werden können. Auf diese Weise lassen sich pharmakologisch nicht verträgliche Säureadditionssalze in pharmakologisch verträgliche Säureadditionssalze umwandeln.

Die Ausgangsverbindungen II und III sind entweder bekannt oder sie können nach bekannten Verfahren hergestellt werden.

Zur Herstellung der Verbindungen der Ausgestaltungen a, b und c bzw. ihrer bevorzugten Vertreter I*, I** und I***geht man von Verbindungen II, II*, II** bzw. II*** und III, III*, III** bzw. III*** aus, in denen die Substituenten und Symbole $R^1$, $R^2$, $R^3$ und A, $R^{1*}$, $R^{2*}$, $R^{3*}$ und A*,

$R^{1**}$, $R^{2**}$, $R^{3**}$ und A** bzw. $R^{1***}$, $R^{2***}$, $R^{3***}$ und A*** die oben angegebenen Bedeutungen haben, wobei die Glieder X, X*, X** bzw. X*** durch geeignete Reste Y bzw. Z, die die oben angegebenen Bedeutungen haben, gebildet werden.

Die folgenden Beispiele erläutern die Erfindung näher ohne sie einzuschränken. Schmp. steht für "Schmelzpunkt", Sdp. für "Siedepunkt".

Beispiele zur Ausführung der Erfindung

1. 5-n-Butylpyridin-2-carbonsäure-(2-diethylaminoethyl)ester

a) 5 g 5-n-Butylpyridin-2-carbonsäure werden unter Außenkühlung mit 20 ml Thionylchlorid übergossen und über Nacht stehengelassen. Die Suspension wird im Vakuum eingedampft, der dunkle Rückstand in 50 ml Acetonitril aufgenommen und zu einer Lösung von 9 g 2-Diethylaminoethanol in 20 ml Acetonitril getropft. Die Mischung wird 6 Stunden bei Raumtemperatur gerührt und anschließend im Vakuum eingedampft. Der halbfeste Rückstand wird in 100 ml 2 n Salzsäure aufgenommen und 3 mal mit je 100 ml Chloroform extrahiert. Die wäßrige Phase wird anschließend mit 2 n Natronlauge auf pH 9 gestellt und das sich abscheidende Öl 3 mal mit je 100 ml Chloroform extrahiert. Die vereinigten Chloroformphasen werden über geglühtem Kaliumcarbonat getrocknet und im Vakuum eingedampft. Das zurückbleibende braune Öl wird einer Kugelrohrdestillation unterworfen. Die Titelverbindung destilliert bei 150°C/0,015 mmHg (2 Pa) als leicht gelbliches Öl.
Ausbeute 3,8 g (53 %).

b) 5,0 g 5-n-Butylpyridin-2-carbonsäuremethylester werden mit 9,1 g 2-Diethylaminoethanol und 100 mg Natrium im Ölbad 2 Stunden auf 130°C erhitzt. Anschließend wird die Mischung im Vakuum eingedampft und wie unter a) beschrieben weiterbehandelt. Nach Destillation erhält man 4,0 g (56 %) der Titelverbindung.

c) 5,0 g 5-n-Butylpyridin-2-carbonsäure werden mit 4,4 g 2-Diethylaminoethanol-hydrochlorid und 1 g p-Toluolsulfonsäure in 50 ml 1,2-Dichlorethan in einem Rundkolben mit Thielepape-Aufsatz 12 Stunden am Rückfluß erhitzt. Das sich im Aufsatz befindende Molekularsieb entfernt das anfallende Wasser. Nach beendeter Reaktion wird der Kolbeninhalt abgekühlt, mit 2 n Salzsäure versetzt und die Dichlorethanphase abgezogen. Die salzsaure, wäßrige Lösung wird wie unter a) beschrieben aufgearbeitet. Man erhält 3,2 g (44 %) der Titelverbindung.

2. 5-n-Butylpyridin-2-carbonsäure-(3-dimethylaminopropyl)-ester

5,0 g 5-n-Butylpyridin-2-carbonsäure werden mit 8,8 g Tetrabutylammoniumhydrogensulfat und 5 g 3-Dimethylaminopropylchlorid-hydrochlorid in einer Mischung von 100 ml 2n Natronlauge und 100 ml Dichlormethan

8 Stunden kräftig bei 30°C gerührt. Anschließend wird die Dichlormethanphase abgetrennt, über Kaliumcarbonat getrocknet und im Vakuum eingedampft. Das zurückbleibende bräunliche Öl wird im Kugelrohr destilliert, wobei die Titelverbindung bei 150°C/0,01 mmHg (1,33 Pa) als leicht gelbliches Öl in einer Ausbeute von 2,0 g (29 %) gewonnen wird.

3. 5-n-Butylpyridin-2-carbonsäure-(3-[4-(2-methoxyphenyl)-piperazinyl-1]-propyl)-ester

1,3 g 5-n-Butylpyridin-2-carbonsäuremethylester und 1,7 g 1-(3-Hydroxypropyl)-4-(2-methoxyphenyl)-piperazin werden bei 140°C unter Zusatz von 100 mg Natrium 16 Stunden gerührt, wobei Methanol langsam abdestilliert wird. Die schwarzbraune Masse wird zur Reinigung an neutralem Kieselgel mit Chloroform/Methanol 98:2 chromatographiert. Man erhält nach Eindampfen der entsprechenden Fraktionen 0,8 g (27 %) der Titelverbindung als hellbraunes Öl.

4. 5-Phenylpyridin-2-carbonsäure-(2-dimethylaminoethyl)ester

5,0 g 5-Phenylpyridin-2-carbonsäuremethylester werden mit 10 ml 2-Dimethylaminoethanol und ca. 100 mg Natrium 3 Stunden auf 50°C und weitere 2 Stunden auf 100°C erhitzt. Anschließend wird das Reaktionsgemisch im Vakuum eingedampft und der dunkelbraune Rückstand wie im Beispiel 3 beschrieben chromatographiert. Man erhält 0,8 g der Titelverbindung als helles, gelblich braunes Öl.

5. 5-Phenylpyridin-2-carbonsäure-(3-diethylaminopropyl)-ester

Aus 5,0 g 5-Phenylpyridin-2-carbonsäuremethylester und 9,3 g 3-Diethylaminopropanol erhält man nach der im Beispiel 1 b beschriebenen Arbeitsweise nach Kugelrohrdestillation bei 200°C/0,15 mmHg (20 Pa) 2,4 g (33 %) der Titelverbindung als bräunliches Öl.

6. 5-Phenylpyridin-2-carbonsäure-(2-[4-phenylpiperazinyl-1]-ethyl)-ester

Aus 5,0 g 5-Phenylpyridin-2-carbonsäuremethylester und 13,8 g 1-(2-Hydroxyethyl)-4-phenylpiperazin in 25 ml Xylol erhält man nach der in Beispiel 3 beschriebenen Arbeitsweise nach chromatographischer Reinigung und Kristallisation aus Petroleumbenzin (Sdp.50-70°C) 0,6 g der Titelverbindung vom Schmp. 150°C.

7. 5-n-Butylpyridin-2-carbonsäure-(2-[4-(2-methoxyphenyl)-piperazinyl-1]-ethyl)-amid

a) 4,66 g 5-n-Butylpyridin-2-carbonsäure werden in 50 ml wasserfreiem Tetrahydrofuran mit 5,9 g Dicyclohexylcarbodiimid 1 Stunde bei Raumtemperatur gerührt. Nach Zugabe von 6,71 g 1-(2-Aminoethyl)-4-(2-methoxyphenyl)-piperazin, gelöst in 30 ml Tetrahydrofuran, wird das Reaktionsgemisch noch 1 Stunde bei Raumtemperatur gerührt und dann 1 Stunde am Rückfluß zum Sieden erhitzt. Man läßt abkühlen, dampft die Reaktionslösung im Vakuum ein und nimmt den Rückstand in 2 n Salzsäure auf. Der zurückbleibende Dicyclohexylharnstoff wird abfiltriert und das Filtrat wird 3 mal mit je 100 ml Chloroform extrahiert. Die wäßrige Phase wird abgetrennt, mit Kaliumcarbonat auf pH 8-9 eingestellt und die sich abscheidende Phase mit Chloroform extrahiert. Die vereinigten Extrakte werden über Kaliumcarbonat sicc. getrocknet und im Vakuum eingedampft. Der Rückstand wird an neutralem Kieselgel mit Chloroform/Methanol 9:1 chromatographiert. Nach Eindampfen der Fraktionen wird das ölige Amid in wenig Aceton aufgenommen, mit 20 ml methanolischer Salzsäure versetzt und im Vakuum vom Lösungsmittel befreit. Nach Trocknung über Kaliumhydroxid bei 90°C im Vakuum erhält man 11,2 g (83 %) der Titelverbindung als Trihydrochlorid vom Schmp. 210°C.

b) 5,0 g 5-n-Butylpyridin-2-carbonsäuremethylester und 12,2 g 1-(2-Aminoethyl)-4-(2-methoxyphenyl)-piperazin werden bei 140°C 2 Stunden gerührt. Nach dem Abkühlen wird das Reaktionsgemisch in Aceton gelöst, mit überschüssiger methanolischer Salzsäure versetzt und eingedampft. Das zurückbleibende Salz wird mehrmals mit Aceton ausgekocht, in Wasser gelöst und 3 mal mit je 30 ml Dichlormethan extrahiert. Die wäßrige Phase wird mit 2 n Natronlauge auf pH 9-10 gebracht und mit Dichlormethan erschöpfend extrahiert. Nach dem Abziehen des Lösungsmittels wird der ölige Rückstand im Kugelrohr bei 260°C/0,15 mmHg (20 Pa) destilliert. Man erhält 6,2 g (85 %) der Titelverbindung als nahezu farbloses Öl.

8. 5-n-Butylpyridin-2-carbonsäure-(3-diethylaminopropyl)-amid

5,0 g 5-n-Butylpyridin-2-carbonsäure werden unter Außenkühlung mit 20 ml Thionylchlorid übergossen und über Nacht stehengelassen. Das Reaktionsgemisch wird im Vakuum eingedampft, der dunkle Rückstand

in Chloroform aufgenommen und zu einer Lösung von 6,7 g 3-Diethylaminopropylamin in 20 ml Chloroform getropft. Die Mischung wird 3 Stunden bei 40°C gerührt und nach dem Abkühlen mit 2 n Natronlauge extrahiert. Die Chloroformphase wird über Kaliumcarbonat getrocknet und eingedampft. Man destilliert den öligen Rückstand im Kugelrohr bei 170°C/0,01 mmHg (1,33 Pa) und erhält 4,2 g (56 %) der Titelverbindung als gelbliches Öl.

9. 5-n-Butylpyridin-2-carbonsäure-(2-dimethylaminoethyl)-amid

5,4 g 5-n-Butylpyridin-2-carbonsäure werden mit 3 g Triethylamin in 60 ml Dichlormethan gelöst. Dazu tropft man unter Eiskühlung 3,3 g Chlorkohlensäureethylester, rührt 15 Minuten bei Raumtemperatur und fügt dem Gemisch 3,4 g 2-Dimethylaminoethylamin zu und rührt weitere 30 Minuten bei Raumtemperatur. Anschließend wird die Dichlormethanlösung mit 2 n Salzsäure ausgeschüttelt; die salzsaure Phase wird mit 2 n Natronlauge alkalisiert und erneut mit Dichlormethan extrahiert. Die vereinigten organischen Extrakte werden mit Wasser extrahiert, über Kaliumcarbonat getrocknet und eingeengt. Nach Destillation im Kugelrohr bei 150°C/0,01 mmHg (1,33 Pa) erhält man 4,9 g (66 %) der Titelverbindung als leicht gelbliches Öl.

10. 5-Phenylpyridin-2-carbonsäure-(3-[4-(2-methoxyphenyl)-piperazinyl-1]-propyl)-amid

5,0 g 5-Phenylpyridin-2-carbonsäuremethylester und 10 g 1-(3-Aminopropyl)-4-(2-methoxyphenyl)-piperazin werden bei 140°C 1,5 Stunden gerührt. Nach dem Abkühlen wird das Reaktionsgemisch in wenig Chloroform aufgenommen und durch Chromatographie an neutralem Kieselgel mit Chloroform/Methanol 95:5 gereinigt. Nach dem Eindampfen der entsprechenden Fraktionen wird der Rückstand aus Petroleumbenzin (Sdp. 50-70°C)/Ethylacetat 9:1 kristallisiert. Man erhält 3 g (30 %) der Titelverbindung vom Schmp. 132-134°C.

11. 5-Phenylpyridin-2-carbonsäure-(3-dimethylaminopropyl)-amid

5,0 g 5-Phenylpyridin-2-carbonsäuremethylester werden in 20 ml 3-Dimethylaminopropylamin 3 Stunden bei 70°C gerührt. Man zieht im Vakuum alle flüchtigen Anteile ab, nimmt den Rückstand in Chloroform auf und chromatographiert an neutralem Kieselgel mit Chloroform/Methanol

9 : 1. Die entsprechenden Fraktionen werden gesammelt, eingeengt und im Kugelrohr bei 210°C/0,1 mmHg (13,3 Pa) destilliert. Man erhält 3,8 g (57 %) der Titelverbindung als gelbliches Öl.

Nach der in Beispiel 11 beschriebenen Arbeitsweise werden aus entsprechend substituierten Pyridin-2-carbonsäuremethylestern und Aminen die folgenden Pyridin-2-carbonsäureamide erhalten:

12. 5-Phenylpyridin-2-carbonsäure-(2-piperidinoethyl)-amid
Aus 5-Phenylpyridin-2-carbonsäuremethylester und 2-Piperidinoethylamin. Schmp. 72-76°C; Ausbeute 44 %.

13. 5-Phenylpyridin-2-carbonsäure-(2-diethylaminoethyl)-amid
Aus 5-Phenylpyridin-2-carbonsäuremethylester und 2-Diethylaminoethylamin. Sdp. (Kugelrohr) 190°C/0,1 mmHg (13,3 Pa); Schmp. 47-53°C; Ausbeute 20 %.

14. 5-(3,4-Dichlorphenyl)-pyridin-2-carbonsäure-(2-[4-(2-methoxyphenyl)-piperazinyl-1]-ethyl)-amid
Aus 5-(3,4-Dichlorphenyl)-pyridin-2-carbonsäuremethylester und 2-[4-(2-Methoxyphenyl)-piperazinyl-1]-ethylamin. Schmp. 104°C; Ausbeute 40 %.

15. 5-(4-Chlorphenyl)-pyridin-2-carbonsäure-(3-[4-(2-methoxyphenyl)-piperazinyl-1]-propyl)-amid
Aus 5-(4-Chlorphenyl)-pyridin-2-carbonsäuremethylester und 3-[4-(2-Methoxyphenyl)-piperazinyl-1]-propylamin . Schmp. 143°C; Ausbeute 83 %.

16. 5-(4-Chlorphenyl)-pyridin-2-carbonsäure-(3-diethylaminopropyl)-amid
Aus 5-(4-Chlorphenyl)-pyridin-2-carbonsäuremethylester und 3-Diethylaminopropylamin. Schmp. 79°C (x 1,5 HCl); Ausbeute 15 %.

17. 5-Cyclohexylmethylpyridin-2-carbonsäure-(3-[4-(2-methoxyphenyl)-piperazinyl-1]-propyl)-amid
Aus 5-Cyclohexylmethylpyridin-2-carbonsäuremethylester und 3-[4-(2-Methoxyphenyl)-piperazinyl-1]-propylamin. Schmp. 186°C (x HCl); Aus-

beute 82 %.

18. 5-(3,4-Dimethoxyphenyl)-pyridin-2-carbonsäure-(3-piperidinopropyl)-amid
Aus 5-(3,4-Dimethoxyphenyl)-pyridin-2-carbonsäuremethylester und 3-Piperidinopropylamin. Schmp. 89°C; Ausbeute 77 %.

19. 5-Cyclohexylmethylpyridin-2-carbonsäure-(3-[4-(2-ethoxyphenyl)-piperazinyl-1]-propyl)-amid
Aus 5-Cyclohexylmethylpyridin-2-carbonsäuremethylester und 3-[4-(2-Ethoxyphenyl)-piperazinyl-1]-propylamin. Schmp. 80°C (x HCl x $H_2O$); Ausbeute 58 %.

20. 5-Cyclohexylmethylpyridin-2-carbonsäure-(3-dimethylaminopropyl)-amid
Aus 5-Cyclohexylmethylpyridin-2-carbonsäuremethylester und 3-Dimethylaminopropylamin. Leicht gelbliches Öl; Ausbeute 62 %.

21. 5-Cyclohexylmethylpyridin-2-carbonsäure-(3-piperidinopropyl)-amid
Aus 5-Cyclohexylmethylpyridin-2-carbonsäuremethylester und 3-Piperidinopropylamin. Schmp. 77°C; Ausbeute 64 %.

22. 5-(3,4-Dimethoxyphenyl)-pyridin-2-carbonsäure-(3-[4-(2-methoxyphenyl)-piperazinyl-1]-propyl)-amid.
Aus 6-(3,4-Dimethoxyphenyl)-pyridin-2-carbonsäuremethylester und 3-[4-(2-Methoxyphenyl)-piperazinyl-1]-propylamin. Schmp. 143°C; Ausbeute 59 %.

23. 5-(3,4-Dimethoxyphenyl)-pyridin-2-carbonsäure-(3-dimethylaminopropyl)-amid
Aus 5-(3,4-Dimethoxyphenyl)-pyridin-2-carbonsäuremethylester und 3-Dimethylaminopropylamin. Schmp. 111°C; Ausbeute 72 %.

24. 5-Cyclohexylmethylpyridin-2-carbonsäure-(3-pyrrolidinopropyl)-amid
Aus 5-Cyclohexylmethylpyridin-2-carbonsäuremethylester und 3-Pyrrolidinopropylamin. Leicht gelbliches Öl; Ausbeute 61 %

25. 5-(3,4-Dimethoxyphenyl)-pyridin-2-carbonsäure-(3-pyrrolidinopropyl)-amid

Aus 5-(3,4-Dimethoxyphenyl)-pyridin-2-carbonsäuremethylester und 3-Pyrrolidinopropylamin. Schmp. 102-104°C; Ausbeute 67 %.

Gewerbliche Anwendbarkeit

Die erfindungsgemäßen substituierten Picolinsäuren der allgemeinen Formel I besitzen wertvolle Eigenschaften, die sie gewerblich verwertbar machen; insbesondere senken sie anhaltend den Blutdruck, wie aus Untersuchungen an wachen, genetisch hypertonen Ratten hervorgeht, wobei sie sich bekannten Picolinsäurederivaten, wie z.B. 5-Butyl-2-pyridincarbonsäure (INN: Fusarsäure) oder 5-Butyl-2-pyridincarbonsäureamid (INN: Bupicomid) überlegen erweisen. Außerdem hemmen die Substanzen die elektrisch induzierte Tachykardie und weisen darüberhinaus eine interessante $\alpha_1$-adrenolytische Wirkung auf.

Die ausgezeichnete Wirksamkeit der erfindungsgemäßen Verbindungen der allgemeinen Formel I gestattet ihren Einsatz in der Humanmedizin, wobei als Indikationen insbesondere primäre (essentielle) und sekundäre Hypertonien aller Schweregrade in Betracht kommen.

Ein weiterer Gegenstand der Erfindung ist daher ein Verfahren zur Behandlung von Säugetieren, die an einer der obengenannten Krankheiten erkrankt sind. Das Verfahren ist dadurch gekennzeichnet, daß man dem erkrankten Säugetier eine therapeutisch wirksame und pharmakologisch verträgliche Menge einer oder mehrerer Verbindungen der allgemeinen Formel I verabreicht.

Gegenstand der Erfindung sind außerdem die Verbindungen der Formel I zur Anwendung bei der Behandlung der Hypertonie.

Ebenso umfaßt die Erfindung die Verwendung von Verbindungen der Formel I bei der Herstellung von Arzneimitteln, die zur Bekämpfung der Hypertonie eingesetzt werden.

Ein weiterer Gegenstand der Erfindung sind Arzneimittel, die eine oder mehrere Verbindungen der allgemeinen Formel I enthalten.

Die Arzneimittel werden nach an sich bekannten, dem Fachmann geläufigen Verfahren hergestellt. Als Arzneimittel werden die erfindungsgemäßen pharmakologisch wirksamen Verbindungen (=Wirkstoffe) entweder als solche, oder vorzugsweise in Kombination mit geeigneten pharmazeutischen Hilfsstoffen in Form von Tabletten, Dragées, Kapseln, Suppositorien, Emulsionen, Suspensionen oder Lösungen eingesetzt.

Welche Hilfsstoffe für die gewünschten Arzneimittelformulierungen geeignet sind, ist dem Fachmann aufgrund seines Fachwissens geläufig. Neben Lösemitteln, Gelbildnern, Salbengrundlagen, Suppositoriengrundlagen, Tabletten-Hilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Die Wirkstoffe können oral oder parenteral appliziert werden, wobei die orale und die intravenöse Applikation bevorzugt ist.

Im allgemeinen hat es sich in der Humanmedizin als vorteilhaft erwiesen, den oder die Wirkstoffe bei oraler Gabe in einer Tagesdosis von etwa 0,01 bis etwa 50, vorzugsweise 0,1 bis 20, insbesondere 0,5 bis 10 mg/kg Körpergewicht, gegebenenfalls in Form mehrerer, vorzugsweise 1 bis 4 Einzelangaben zur Erzielung des gewünschten Ergebnisses zu verabreichen. Bei einer parenteralen, z.B. intravenösen Behandlung können ähnliche Dosierungen zur Anwendung kommen. Bei einschleichender Dosierung wird zu Beginn der Behandlung eine geringere Dosis verabreicht, dann langsam auf eine höhere Dosis übergegangen. Nach Erreichen der gewünschten Blutdrucksenkung wird wieder auf eine niedrigere Dosis zurückgegangen.

Die Festlegung der jeweils erforderlichen optimalen Dosierung und Applikationsart der Wirkstoffe kann durch jeden Fachmann aufgrund seines Fachwissens leicht erfolgen.

Sollen die erfindungsgemäßen substituierten Picolinsäuren und/oder ihre Säureadditionssalze zur Behandlung der Hypertonie eingesetzt werden, so können die pharmazeutischen Zubereitungen auch einen oder mehrere andere pharmakologisch aktive Bestandteile anderer Arzneimittelgruppen, wie andere Antihypertensiva, β-Rezeptorenblocker, Diuretika, Saluretika, Alkaloide, etc., wie Dihydralazin, Propranolol, Labetalol, Mefrusid, Clopamid, Spironolacton, Chlorthalidon, Furosemid, Polythiazid, Hydrochlorothiazid, Reserpin, Dihydroergocristin, Rescinnamin, Rauwolfia-Gesamtalkaloide etc. enthalten.

Die Formulierung der Wirkstoffe kann z.B. auf folgende Weise erfolgen:

a) Tabletten mit einem Wirkstoffgehalt von 20 mg

10,81 kg 5-Cyclohexylmethylpyridin-2-carbonsäure-(3-[4-(2-methoxyphenyl)-piperazinyl-1]-propyl)-amid-hydrochlorid, 50,0 g Calciumsulfatdihydrat, 31,0 kg Maisstärke und 3,0 kg Polyvinylpyrrolidon werden mit 20 l Wasser befeuchtet und durch ein Sieb mit 1,25 mm Maschenweite granuliert. Das Granulat wird im Wirbelschichttrockner bis zu einer relativen Feuchte von 50-60 % getrocknet und dann mit 8,0 g Natriumcarboxymethylcellulose, 2,0 kg Talkum und 1,0 kg Magnesiumstearat versetzt. Das fertige Granulat wird zu Tabletten à 200 mg und 8 mm Durchmesser verpreßt.

b) Ampullen mit einem Wirkstoffgehalt von 22,5 mg

1,502 kg 5-(4-Chlorphenyl)-pyridin-2-carbonsäure-(3-diethylaminopropyl)-amid-fumarat werden in 80 Liter aqua bidestillata gelöst und mit 4,337 kg Mannit versetzt. Die Lösung wird mit aqua bidestillata auf 100 kg aufgefüllt, über einen Filter sterilfiltriert und in 2-ml-Ampullen abgefüllt.

c) 25000 Retard-Kapseln mit einem Wirkstoffgehalt von 30 mg

8,9 kg 5-(4-Chlorphenyl)-pyridin-2-carbonsäure-(3-[4-(2-methoxyphenyl)-piperazinyl-1]-propyl)-amid-hydrochlorid und 3,1 kg Hydroxypropylmethylcellulose werden in einem Gemisch von 16 l 96 %igem Ethanol und 24 l

Wasser gelöst. In diese Lösung wird 1,1 kg Talkum suspendiert. In einem Wirbelschichtgerät werden 16 kg Zuckerpellets mit dieser Wirkstofflösung besprüht. Die erhaltenen Wirkstoffpellets werden im gleichen Gerät mit einer Lösung aus 0,6 kg Ethylcellulose, 0,3 kg Hydroxypropylmethylcellulose und 0,12 kg Stearinsäure in 14,8 l Methylenchlorid und 4,0 l 96 %igem Ethanol besprüht. Nach dem Trocknen der überzogenen Wirkstoffpellets werden diese in Hartgelatinekapseln der Größe 4 abgefüllt.

Pharmakologie

Zur Bestimmung der antihypertensiven Wirkung werden die unten aufgeführten Verbindungen in den angegebenen Dosen an vier aufeinander folgenden Tagen an je 6 Ratten (Stamm SHR/N/Ibm/Bm, ♂, 250-350 g) mit genetisch bedingtem Hochdruck (RR > 180 mmHg) täglich einmal mittels Schlundsonde verabfolgt. Die Messung des Blutdrucks erfolgt jeweils 2, 6 und gegebenenfalls 24 Stunden nach Substanzgabe.

Die Blutdruckmessung wird in einer Wärmekammer bei 36°C vorgenommen, um eine bessere Durchblutung der Schwanzarterie zu erreichen. Hierzu werden die Tiere in perforierte Lochblechkäfige verbracht und 20 - 40 Min. nach Beginn der Aufwärmung gemessen. Zur Messung des systolischen arteriellen Drucks wird eine ringförmige Manschette mit aufblasbarer Gummimembran zur Unterbindung der Durchblutung und ein ringförmiger Piezokristallaufnehmer zur Erfassung der Pulswellen auf den Schwanz aufgeschoben. Nach erfolgter Unterbindung des Blutstroms in der Schwanzarterie wird der Manschettendruck kontinuierlich reduziert. Die Wiederkehr der Pulswellen bei Druckablassen wird automatisch als systolischer Blutdruck erkannt und ausgedruckt (Bühler,R. et al.: Microprocessor-based automation of blood pressure measurement in the conscious rat. Proceedings of the 4th international symposium on rats with spontaneous hypertension and related studies, Rascher,R. et al. (Eds.), Schattauer Verlag, Stuttgart, New York, 1982, S.410-413). Pulssignale und Druckverlauf werden zur Auswertung graphisch aufgezeichnet.

Zur Gewöhnung an den Meßvorgang werden die Tiere vor Substanzprüfung 14 Tage traniniert. In der zweiten Trainingswoche werden Blutdruck-Vorwerte erhoben. Tiergruppen, die Substanz erhalten, werden gegen eine Kontrollgruppe geprüft.

Zur Prüfung der Hemmung der elektrisch induzierten Tachykardie wird nach Kobinger und Pichler vorgegangen (Kobinger, W. und Pichler, L.: Investigation into different types of post- and presynaptic α-adrenoceptors at cardiovascular sites in rats. Eur.J.Pharmacol. 65, 393-402, 1980). Zur Prüfung der $\alpha_1$-adrenolytischen Wirkung wird das Modell nach Arunlakshana und Schild eingesetzt (Arunlakshana, O. und Schild, H.O.: Some quantitative uses of drug antagonists. Brit.J.Pharmacol. 14, 48, 1959).

Die Toxizitätsuntersuchungen werden an weiblichen NMRI-Mäusen (Körpergewicht 22 - 26 g) durchgeführt. Die Tiere (5 Tiere pro Dosis) erhalten Futter und Wasser ad libitum. Verschiedene Dosen der Substanzen werden oral verabreicht. Die Beobachtungsdauer beträgt 14 Tage. Die $LD_{50}$, d.h. die Dosis bei der 50 % der Tiere sterben, wird aus der Dosiswirkungskurve graphisch ermittelt.

In den anschließenden Tabellen werden die untersuchten Verbindungen durch laufende Nummern gekennzeichnet, die wie folgt zugeordnet sind:

| lfd. Nr. | Name der Verbindung |
|---|---|
| 1 | 5-Butyl-2-pyridincarbonsäure (Fusarsäure) |
| 2 | 5-Butyl-2-pyridincarbonsäureamid (Bupicomid) |
| 3 | 5-n-Butylpyridin-2-carbonsäure-(3-dimethylaminopropyl)-ester |
| 4 | 5-Phenylpyridin-2-carbonsäure-(3-diethylaminopropyl)-ester |
| 5 | 5-Phenylpyridin-2-carbonsäure-(3-[4-(2-methoxyphenyl)-piperazinyl-1]-propyl)-amid |
| 6 | 5-Phenylpyridin-2-carbonsäure-(3-dimethylaminopropyl)-amid |
| 7 | 5-Phenylpyridin-2-carbonsäure-(2-diethylaminoethyl)-amid |
| 8 | 5-(4-Chlorphenyl)-pyridin-2-carbonsäure-(3-[4-(2-methoxyphenyl)-piperazinyl-1]-propyl)-amid |
| 9 | 5-(4-Chlorphenyl)-pyridin-2-carbonsäure-(3-diethylamino-propyl)-amid |
| 10 | 5-Cyclohexylmethylpyridin-2-carbonsäure-(3-[4-(2-methoxyphenyl)-piperazinyl-1]-propyl)-amid |
| 11 | 5-Cyclohexylmethylpyridin-2-carbonsäure-(3-[4-(2-ethoxyphenyl)-piperazinyl-1]-propyl)-amid |
| 12 | 5-Cyclohexylmethylpyridin-2-carbonsäure-(3-piperidino-propyl)-amid |

Tabelle I gibt für die Vertreter der erfindunggemäßen Verbindungen die prozentuale Senkung des Blutdrucks nach oraler Verabreichung bei der Ratte und die letale Wirkung nach oraler Gabe bei der Maus wieder.

Tabelle I

%-Änderungen des Blutdrucks (BP) an genetisch hypertonen Ratten nach täglich einmaliger p.o.-Applikation an vier aufeinanderfolgenden Tagen (N=6/Dosis) und Toxizität (p.o.;$LD_{50}$) an Mäusen (N=5/Dosis).

| lfd. Nr. | Dosis (mg/kg) | Dosis (µMol/kg) | BP (%-Änderungen) 1. Tag 2h | 1. Tag 6h | 1. Tag 24h | 2.Tag 6h | 3.Tag 6h | 3.Tag 24h | 4.Tag 2h | 4.Tag 6h | $LD_{50}$p.o. (mg/kg) |
|---|---|---|---|---|---|---|---|---|---|---|---|
| 1 | 6,4 | 35,7 | -4 | +3 | -1 | +3 | -6 | +5 | -1 | -3 | |
| | 20,0 | 111,6 | -8 | -5 | | -6 | +2 | | -9 | -6 | 260 |
| | 25,6 | 142,8 | +15 | -3 | 0 | -10 | -15 | -5 | -5 | -10 | |
| 2 | 20,0 | 112,2 | -5 | -10 | | -6 | -4 | | -5 | -7 | |
| | 20,0 | 112,2 | -8 | -13 | 0 | -9 | -7 | -2 | -8 | -10 | |
| 3 | 18,5 | 70,0 | -10 | -9 | -2 | -16 | -19 | -1 | -13 | -15 | |
| | 9,25 | 35,0 | -2 | -3 | -3 | -9 | -10 | -2 | -9 | -10 | |
| 4 | 21,9 | 70,0 | -8 | -6 | -4 | -5 | -8 | 0 | -7 | -7 | |
| | 10,9 | 35,0 | -8 | -4 | -5 | -4 | -8 | -3 | -12 | -8 | |
| 5 | 20,0 | 46,5 | -3 | -2 | -6 | -8 | -14 | -5 | -6 | -9 | |
| | 10,0 | 23,2 | -4 | -10 | -6 | -10 | -13 | -12 | -13 | -12 | |
| 6 | 6,4 | 22,6 | -7 | -7 | -2 | -8 | -8 | -6 | -8 | -9 | |
| | 25,6 | 90,3 | +1 | -7 | +4 | -8 | -7 | -4 | -7 | -10 | |
| 7 | 20,8 | 70,0 | -5 | -9 | -7 | -7 | -10 | -6 | -6 | -7 | |
| | 10,4 | 35,0 | -7 | -6 | -4 | -8 | -9 | -4 | -4 | -4 | |
| 8 | 6,4 | 13,8 | -5 | -9 | -5 | -10 | -7 | -10 | -10 | | |
| | 11,6 | 25,0 | -7 | +1 | -6 | -15 | -19 | -23 | -14 | -20 | ≫1000 |
| | 25,6 | 55,1 | -6 | -9 | -8 | -9 | -9 | -10 | -6 | | |
| | 46,5 | 100,0 | -7 | +9 | -11 | -21 | -27 | -21 | -22 | -13 | |

Fortsetzung Tabelle I:

| lfd. Nr. | Dosis (mg/kg) | Dosis (µMol/kg) | BP (% Änderungen) 1.Tag 2h | 1.Tag 6h | 1.Tag 24h | 2.Tag 6h | 3.Tag 6h | 3.Tag 24h | 4.Tag 2h | 4.Tag 6h | $LD_{50}$ (p.o.) mg/kg |
|---|---|---|---|---|---|---|---|---|---|---|---|
| 9 | 6,4 | 18,5 | -6 | -4 | -6 | -19 | -5 | -1 | -9 | -14 | > 1000 |
| | 25,6 | 74,0 | -19 | -11 | -22 | -20 | -21 | -15 | -19 | -25 | |
| 10 | 6,4 | 14,2 | -19 | -14 | -20 | -12 | -11 | -5 | -16 | -24 | ≫ 1000 |
| | 25,6 | 56,8 | -26 | -20 | -14 | -12 | -12 | -3 | -12 | -22 | |
| 11 | 6,4 | 13,8 | +2 | -1 | +10 | -14 | -10 | +4 | | -10 | |
| | 25,6 | 55,1 | -7 | -9 | +4 | +3 | -15 | -10 | | | |
| 12 | 6,4 | 18,6 | -14 | -7 | -1 | -1 | -6 | -1 | -16 | -10 | |
| | 25,6 | 74,5 | -10 | -12 | -10 | -2 | -11 | -6 | -11 | -10 | |

Die folgende Tabelle II gibt die Therapeutische Wirksamkeit (Th.W.) der in Tabelle I aufgeführten Verbindungen wieder. Die Therapeutische Wirksamkeit ist der Quotient aus maximaler prozentualer Änderung des Blutdrucks BP und der applizierten Dosis (µMol/kg), wobei jeweils der Mittelwert dieses Quotienten für die in Tabelle I angegebenen Dosierungen angegeben wird.

Tabelle II

Therapeutische Wirksamkeit (Th.W.) errechnet aus der maximalen prozentualen Änderung des Blutdrucks (BP) und der applizierten Dosis.

| lfd. Nr. | Th.W. $\left[\frac{\text{\%-Änderung BP}}{\mu mol/kg} \times 100\right]$ 1. Tag 2h | 1. Tag 6h | 1. Tag 24h | 2. Tag 6h | 3. Tag 6h | 3. Tag 24 h | 4. Tag 2h | 4. Tag 6h |
|---|---|---|---|---|---|---|---|---|
| 1 | -2,6 | 0,6 | -1,4 | -1,3 | -8,5 | 5,2 | -4,8 | -6,9 |
| 2 | -5,6 | -10,3 | 1,3 | -6,7 | -4,5 | 0,4 | -5,6 | -7,6 |
| 3 | -10,0 | -10,7 | -5,7 | -24,3 | -27,9 | -3,6 | -22,1 | -25,0 |
| 4 | -17,1 | -10,0 | -10,0 | -9,3 | -17,1 | -4,3 | -22,1 | -16,4 |
| 5 | -11,8 | -23,7 | -19,4 | -30,2 | -43,1 | -31,2 | -34,5 | -35,5 |
| 6 | -14,9 | -19,4 | -2,2 | -22,1 | -21,6 | -15,5 | -21,6 | -25,4 |
| 7 | -13,6 | -15,0 | -10,7 | -16,4 | -20,0 | -7,8 | -10,0 | -10,7 |

Fortsetzung Tabelle II:

| lfd. Nr. | Th.W. [ %-Änderung BP / µmol/kg x 100 ] | | | | | | | |
|---|---|---|---|---|---|---|---|---|
| | 1. Tag | | | 2. Tag | 3. Tag | | 4. Tag | |
| | 2h | 6h | 24h | 6h | 6h | 24 h | 2h | 6h |
| 8 | -20,5 | -17,1 | -21,4 | -42,4 | -42,5 | -50,9 | -40,3 | -46,5 |
| 9 | -29,1 | -18,3 | -31,1 | -64,9 | -27,7 | -12,9 | -37,2 | -54,8 |
| 10 | -89,8 | -66,9 | -82,7 | -52,8 | -49,3 | -20,3 | -66,9 | -103,9 |
| 11 | 0,9 | -11,8 | 39,9 | -48,0 | -49,9 | 23,6 | -- | -36,2 |
| 12 | -44,4 | -26,9 | -9,4 | -4,1 | -23,6 | -6,8 | -50,4 | -33,6 |

In der Tabelle III ist der Therapeutische Index mit der Normierung Th.W. Fusarsäure = 1 für die in Tabelle II angegebenen Tage wieder gegeben. Die 2h-, 6h- und 24 h-Werte des 1., die 6 h- und 24h-Werte des 3. und die 2 h- und 6h - Werte des 4. Tages wurden hierbei gemittelt.

Tabelle III

Therapeutischer Indes (Th.I.) mit der Normierung Th.W. Fusarsäure =1.

| lfd. Nr. | Th.I. Fusarsäure = 1 | | | |
|---|---|---|---|---|
| | 1. Tag | 2. Tag | 3. Tag | 4. Tag |
| 1 | 1 | 1 | 1 | 1 |
| 2 | 4,3 | 5,2 | 2,5 | 1,1 |
| 3 | 7,8 | 18,7 | 9,5 | 4,1 |
| 4 | 10,9 | 7,2 | 6,5 | 3,3 |
| 5 | 16,2 | 23,2 | 22,5 | 6,0 |
| 6 | 10,8 | 17,0 | 11,2 | 4,1 |
| 7 | 11,6 | 12,6 | 8,4 | 1,8 |
| 8 | 17,4 | 32,6 | 28,3 | 7,5 |
| 9 | 23,1 | 49,9 | 12,3 | 7,9 |
| 10 | 70,6 | 40,6 | 21,1 | 14,7 |
| 11 | -25,7 | 36,9 | 8,0 | 5,2 |
| 12 | 23,8 | 3,2 | 9,2 | 7,2 |

## Patentansprüche

(für alle benannten Vertragsstaaten außer Österreich)

1. Verbindungen der allgemeinen Formel I

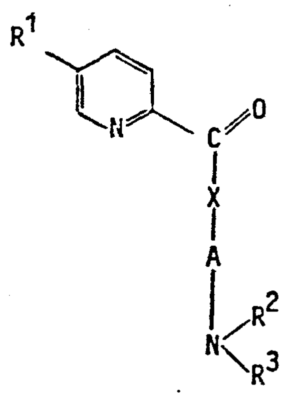

(I),

worin

$R^1$ Alkyl mit 1 bis 7 Kohlenstoffatomen - jedoch nicht Butyl, wenn $R^2$ und $R^3$ die Bedeutung Alkyl haben und X NH bedeutet -, Cycloalkylalkyl mit 3 bis 7 Kohlenstoffatomen im Cycloalkyl- und 1 bis 4 Kohlenstoffatomen im Alkylenteil, Phenyl oder substituiertes Phenyl mit bis zu drei Substituenten aus der Gruppe Halogen, Hydroxy, Alkyl mit 1 bis 4 Kohlenstoffatomen und Alkoxy mit 1 bis 4 Kohlenstoffatomen bedeutet,

$R^2$ Alkyl mit 1 bis 4 Kohlenstoffatomen oder Alkenyl mit 3 bis 5 Kohlenstoffatomen bedeutet,

$R^3$ Alkyl mit 1 bis 4 Kohlenstoffatomen oder Alkenyl mit 3 bis 5 Kohlenstoffatomen bedeutet, oder

$R^2$ und $R^3$ gemeinsam und unter Einschluß des Stickstoffatoms, an das sie gebunden sind, Pyrrolidino, Morpholino, Piperidino, Hexahydroazepino oder in 4-Position durch $R^4$ substituiertes 1-Piperazinyl bedeuten,

$R^4$ Wasserstoff, Alkyl mit 1 bis 4 Kohlenstoffatomen, Phenyl, Benzyl oder substituiertes Phenyl mit bis zu drei Substituenten aus der Gruppe Halogen, Hydroxy, Alkyl mit 1 bis 4 Kohlenstoffatomen und Alkoxy mit 1 bis 4 Kohlenstoffatomen bedeutet,

A geradkettiges oder verzweigtes Alkylen mit 1 bis 5 Kohlenstoffatomen

und

X O (Sauerstoff) oder NH bedeutet,

sowie die Säureadditionssalze dieser Verbindungen, wobei $R^1$, $R^2$ und $R^3$ nicht gleichzeitig Alkyl bedeuten, wenn X O(Sauerstoff) und A Ethylen darstellt.

2. Verbindungen der allgemeinen Formel I*

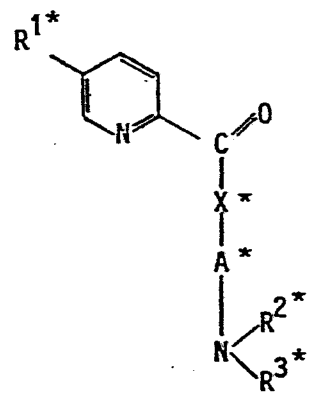

(I*),

worin

$R^{1*}$ Alkyl mit 1 bis 5 Kohlenstoffatomen (jedoch nicht Butyl, wenn $R^{2*}$ und $R^{3*}$ die Bedeutung Methyl oder Ethyl haben und X NH darstellt) bedeutet,

$R^{2*}$ Methyl oder Ethyl bedeutet,

$R^{3*}$ Methyl oder Ethyl bedeutet oder

$R^{2*}$ und $R^{3*}$ gemeinsam und unter Einschluß des Stickstoffatoms, an das sie gebunden sind, Pyrrolidino, Piperidino oder in 4-Position durch $R^{4*}$ substituiertes 1-Piperazinyl bedeuten,

$R^{4*}$ Phenyl oder durch Methoxy oder Ethoxy substituiertes Phenyl bedeutet,

A* Ethylen oder Trimethylen und

X* O(Sauerstoff) oder NH bedeutet,

sowie die Säureadditionssalze dieser Verbindungen, wobei $R^{2*}$ und $R^{3*}$ nicht Methyl oder Ethyl bedeuten, wenn X* O(Sauerstoff) und A* Ethylen darstellt.

3. Verbindungen der allgemeinen Formel I**

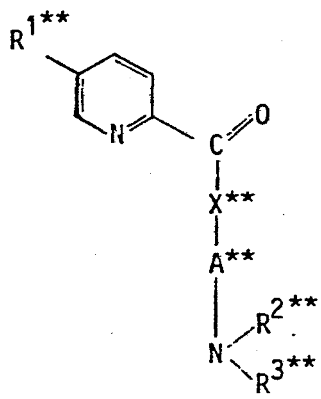

(I**),

worin

R1** Cycloalkylalkyl mit 5 oder 6 Kohlenstoffatomen im Cycloalkyl- und 1 oder 2 Kohlenstoffatomen im Alkylenteil, Phenyl oder substituiertes Phenyl mit bis zu zwei Substituenten aus der Gruppe Chlor, Methoxy oder Ethoxy bedeutet,

R2** Methyl oder Ethyl bedeutet,

R3** Methyl oder Ethyl bedeutet,

A** Ethylen oder Trimethylen und

X** O(Sauerstoff) oder NH bedeutet,

sowie die Säureadditionssalze dieser Verbindungen.

4. Verbindungen der allgemeinen Formel I***

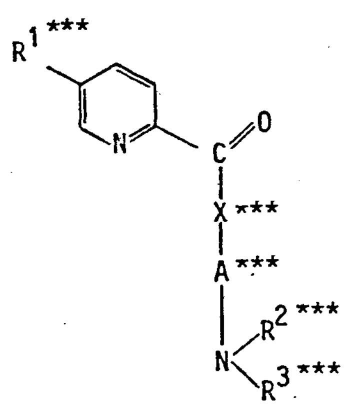

(I***),

worin

$R^{1***}$ Cycloalkylalkyl mit 5 oder 6 Kohlenstoffatomen im Cycloalkyl- und 1 oder 2 Kohlenstoffatomen im Alkylenteil, Phenyl oder substituiertes Phenyl mit bis zu zwei Substituenten aus der Gruppe Chlor, Methoxy oder Ethoxy bedeutet,

$R^{2***}$ und $R^{3***}$ gemeinsam und unter Einschluß des Stickstoffatoms, an das sie gebunden sind, Pyrrolidino, Piperidino oder in 4-Position durch $R^{4***}$ substituiertes 1-Piperazinyl bedeuten,

$R^{4***}$ Wasserstoff, Methyl, Phenyl oder durch Methoxy oder Ethoxy substituiertes Phenyl bedeutet,

A*** Ethylen oder Trimethylen und

X*** O(Sauerstoff) oder NH bedeutet,

sowie die Säureadditionssalze dieser Verbindungen.

5. Verfahren zur Herstellung von Verbindungen der Formel I nach Anspruch 1, dadurch gekennzeichnet, daß man Picolinsäurederivate der allgemeinen Formel II

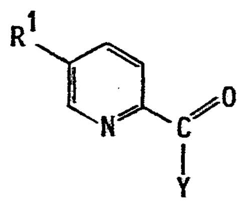

(II),

mit Aminen der allgemeinen Formel III

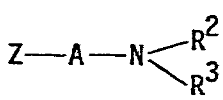

(III),

umsetzt und gewünschtenfalls anschließend in die Säureadditionssalze überführt, wobei die Reste Y und Z so zu wählen sind, daß das Glied X gebildet wird, und wobei $R^1$, $R^2$, $R^3$, A und X die in Anspruch 1 angegebenen Bedeutungen haben.

6. Verbindungen nach einem oder mehreren der Ansprüche 1, 2, 3 oder 4 zur Anwendung bei der Behandlung des Bluthochdrucks.

7. Arzneimittel enthaltend eine oder mehrere Verbindungen nach einem oder mehreren der Ansprüche 1, 2, 3 oder 4 und/oder ihre pharmakologisch verträglichen Säureadditionssalze.

## Patentansprüche

(für Österreich)

1. Verfahren zur Herstellung von Verbindungen der allgemeinen Formel I

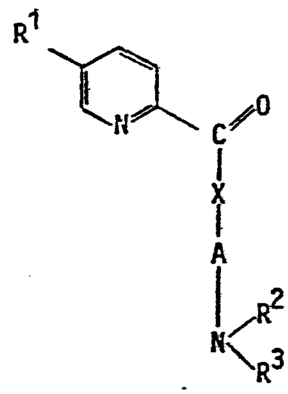

(I),

worin

$R^1$ Alkyl mit 1 bis 7 Kohlenstoffatomen - jedoch nicht Butyl, wenn $R^2$ und $R^3$ die Bedeutung Alkyl haben und X NH bedeutet -, Cycloalkylalkyl mit 3 bis 7 Kohlenstoffatomen im Cycloalkyl- und 1 bis 4 Kohlenstoffatomen im Alkylenteil, Phenyl oder substituiertes Phenyl mit bis zu drei Substituenten aus der Gruppe Halogen, Hydroxy, Alkyl mit 1 bis 4 Kohlenstoffatomen und Alkoxy mit 1 bis 4 Kohlenstoffatomen bedeutet,

$R^2$ Alkyl mit 1 bis 4 Kohlenstoffatomen oder Alkenyl mit 3 bis 5 Kohlenstoffatomen bedeutet,

$R^3$ Alkyl mit 1 bis 4 Kohlenstoffatomen oder Alkenyl mit 3 bis 5 Kohlenstoffatomen bedeutet, oder

$R^2$ und $R^3$ gemeinsam und unter Einschluß des Stickstoffatoms, an das sie gebunden sind, Pyrrolidino, Morpholino, Piperidino, Hexahydroazepino oder in 4-Position durch $R^4$ substituiertes 1-Piperazinyl bedeuten,

$R^4$ Wasserstoff, Alkyl mit 1 bis 4 Kohlenstoffatomen, Phenyl, Benzyl oder substituiertes Phenyl mit bis zu drei Substituenten aus der Gruppe Halogen, Hydroxy, Alkyl mit 1 bis 4 Kohlenstoffatomen und Alkoxy mit 1 bis 4 Kohlenstoffatomen bedeutet,

A geradkettiges oder verzweigtes Alkylen mit 1 bis 5 Kohlenstoffatomen und

X 0 (Sauerstoff) oder NH bedeutet,
sowie den Säureadditionssalzen dieser Verbindungen, wobei $R^1$, $R^2$ und $R^3$ nicht gleichzeitig Alkyl bedeuten, wenn X O(Sauerstoff) und A Ethylen darstellt, dadurch gekennzeichnet, daß man Picolinsäurederivate der allgemeinen Formel II

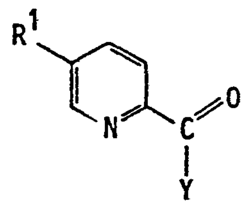

(II),

mit Aminen der allgemeinen Formel III

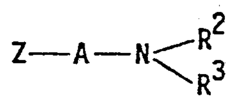

(III),

umsetzt und gewünschtenfalls anschließend in die Säureadditionssalze überführt, wobei die Reste Y und Z so zu wählen sind, daß das Glied X gebildet wird, und wobei $R^1$, $R^2$, $R^3$, A und X die vorstehend angegebenen Bedeutungen haben.

2. Verfahren zur Herstellung von Verbindungen der allgemeinen Formel I*

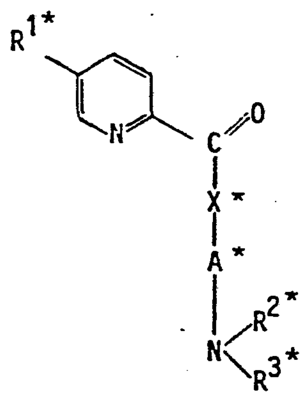

(I*),

worin
$R^{1*}$ Alkyl mit 1 bis 5 Kohlenstoffatomen (jedoch nicht Butyl, wenn $R^{2*}$ und $R^{3*}$ die Bedeutung Methyl oder Ethyl haben und X NH darstellt) bedeutet,

$R^{2*}$ Methyl oder Ethyl bedeutet,

$R^{3*}$ Methyl oder Ethyl bedeutet oder

$R^{2*}$ und $R^{3*}$ gemeinsam und unter Einschluß des Stickstoffatoms, an das sie gebunden sind, Pyrrolidino, Piperidino oder in 4-Position durch $R^{4*}$ substituiertes 1-Piperazinyl bedeuten,

$R^{4*}$ Phenyl oder durch Methoxy oder Ethoxy substituiertes Phenyl bedeutet,

A* Ethylen oder Trimethylen und

X* O(Sauerstoff) oder NH bedeutet,

sowie den Säureadditionssalzen dieser Verbindungen, wobei $R^{2*}$ und $R^{3*}$ nicht Methyl oder Ethyl bedeuten, wenn X* O(Sauerstoff) und A* Ethylen darstellt, dadurch gekennzeichnet, daß man Picolinsäurederivate der allgemeinen Formel II*

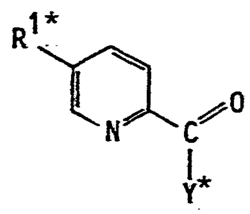

(II*)

mit Aminen der allgemeinen Formel III*

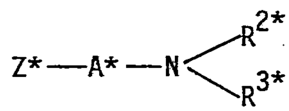

(III*)

umsetzt und gewünschtenfalls anschließend in die Säureadditionssalze überführt, wobei die Reste Y* und Z* so zu wählen sind, daß das Glied X* gebildet wird, wobei $R^{1*}$, $R^{2*}$, $R^{3*}$, A* und X* die vorstehend angegebenen Bedeutungen haben.

3. Verfahren zur Herstellung von Verbindungen der allgemeinen Formel I**

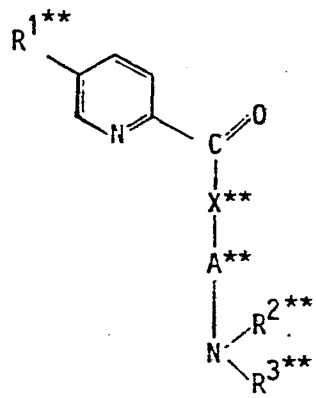

(I**),

worin

R1** Cycloalkylalkyl mit 5 oder 6 Kohlenstoffatomen im Cycloalkyl- und 1 oder 2 Kohlenstoffatomen im Alkylenteil, Phenyl oder substituiertes Phenyl mit bis zu zwei Substituenten aus der Gruppe Chlor, Methoxy oder Ethoxy bedeutet,

R2** Methyl oder Ethyl bedeutet,

R3** Methyl oder Ethyl bedeutet,

A** Ethylen oder Trimethylen und

X** O(Sauerstoff) oder NH bedeutet,

sowie den Säureadditionssalzen dieser Verbindungen, dadurch gekennzeichnet, daß man Picolinsäurederivate der allgemeinen Formel II**

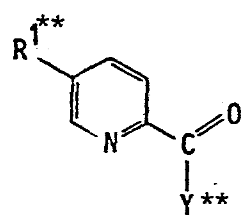

(II**),

mit Aminen der allgemeinen Formel III**

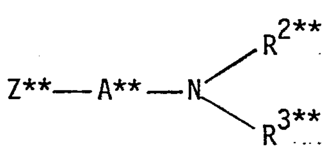

(III**),

umsetzt und gewünschtenfalls anschließend in die Säureadditionssalze überführt, wobei die Reste Y** und Z** so zu wählen sind, daß das Glied X** gebildet wird, und wobei R1**, R2**, R3**, A** und X** die vorstehend angegebenen Bedeutungen haben.

4. Verfahren zur Herstellung von Verbindungen der allgemeinen Formel I***

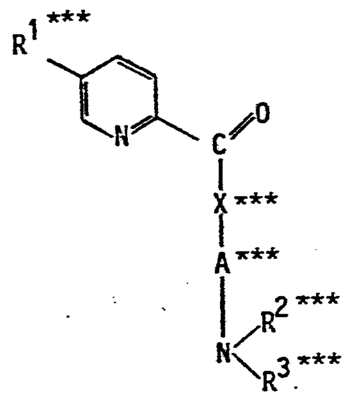

(I***),

worin

R1*** Cycloalkylalkyl mit 5 oder 6 Kohlenstoffatomen im Cycloalkyl- und 1 oder 2 Kohlenstoffatomen im Alkylenteil, Phenyl oder substituiertes Phenyl mit bis zu zwei Substituenten aus der Gruppe Chlor, Methoxy oder Ethoxy bedeutet,

R2*** und R3*** gemeinsam und unter Einschluß des Stickstoffatoms, an das sie gebunden sind, Pyrrolidino, Piperidino oder in 4-Position durch R4*** substituiertes 1-Piperazinyl bedeuten,

R4*** Wasserstoff, Methyl, Phenyl oder durch Methoxy oder Ethoxy substituiertes Phenyl bedeutet,

A*** Ethylen oder Trimethylen und

X*** O(Sauerstoff) oder NH bedeutet,

sowie den Säureadditionssalzen dieser Verbindungen, dadurch gekennzeichnet, daß man Picolinsäurederivate der allgemeinen Formel II***

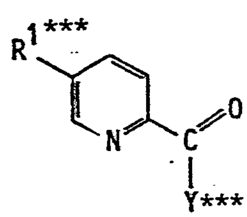

(II***),

mit Aminen der allgemeinen Formel III***

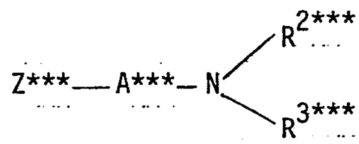

(III***),

umsetzt und gewünschtenfalls anschließend in die Säureadditionssalze überführt, wobei die Reste Y*** und Z*** so zu wählen sind, daß das Glied X*** gebildet wird, und wobei $R^{1***}$, $R^{2***}$, $R^{3***}$, A*** und X*** die vorstehend angegebenen Bedeutungen haben.

0123700

Europäisches Patentamt

**EUROPÄISCHER RECHERCHENBERICHT**

Nummer der Anmeldung

EP 83 10 4094

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| Y | IL FARMACO, Editione Scientif., Band 36, Nr. 4, 1981, Seiten 260-68, Milano (IT); C.RUNTI et al.: "Fusaric acid derivatives and analogues as possible antihypertensive drugs". *Seite 263, Tabelle 1; Seite 266* --- | 1,5-7 | C 07 D 213/79<br>C 07 D 213/81<br>A 61 K 31/44 |
| Y | CHEMICAL ABSTRACTS, Band 90, Nr. 1, 1. Januar 1979, Seite 567, Nr. 6197z, Columbus Ohio (USA); T.M.MEDVEDEVA et al.: "Synthesis and pharmacological activity of fusaric acid and its derivatives". & KHIM. FARM. ZH. 1978, 12(5), 66-9. --- | 1,5-7 | |
| Y,D | SOVIET INVENTIONS ILLUSTRATED, Section Chemical, Derwent Publications Ltd., Woche B06, 21. März 1979, Seite 74; & SU - A - 598 892 (AS USSR BIOCH PHYSI) (17.03.78) *Zusammenfassung* --- | 1,5-7 | RECHERCHIERTE SACHGEBIETE (Int. Cl. 3)<br>C 07 D 213/00<br>A 61 K 31/00 |
| Y | US-A-4 009 174 (R.CLUZAN) *Insgesamt* --- | 1,5-7 | |
| E | DE-A-3 239 573 (BYK GULDEN LOMBERG) *Insgesamt* ----- | 1-7 | |

Der vorliegende Recherchenbericht wurde für alle Patentansprüche erstellt.

| Recherchenort | Abschlußdatum der Recherche | Prüfer |
|---|---|---|
| DEN HAAG | 06-12-1983 | NUYTS A.M.K.A. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument